# EUROPÄISCHE PATENTSCHRIFT

(11) **EP 0 525 579 B1**
(45) Veröffentlichungstag und Bekanntmachung des Hinweises auf die Patenterteilung: **20.09.1995**
(21) Anmeldenummer: 92112404.6
(22) Anmeldetag: 20.07.1992
(51) Int. Cl.: C07C 57/26, C07C 51/08, C07C 51/09, C07C 255/31

(54) **Verfahren zur Herstellung von substituierten Hexensäuren**
Process for preparing substituted hexemic acids
Procédé de préparation d'acides hexémiques substitués

(30) Priorität: 02.08.1991 CH 2308/91
(43) Veröffentlichungstag der Anmeldung: 03.02.1993
(73) Patentinhaber: GIVAUDAN-ROURE (INTERNATIONAL) S.A., CH-1214 Vernier, Genève (CH)
(72) Erfinder: Helmlinger, Daniel, CH-8600 Dübendorf (CH)
(74) Vertreter: Urech, Peter, Dr.

(56) Entgegenhaltungen:
- EP-A- 0 150 290
- 'ORGANICUM' 1988 , VEB DEUTSCHER VERLAG DER WISSENSCHAFT , BERLIN 17 AUFLAGE
- AGRICULTURAL AND BIOLOGICAL CHEMISTRY Bd. 50, Nr. 6, 1986, TOKYO,JP Seiten 1475 - 1480 MATSUI M ET AL 'New synthesis of ambrox,ambra oxide and their stereoisomers'
- JOURNAL OF ORGANIC CHEMISTRY Bd. 27, 1962, WASHINGTON D.C. Seiten 3505 - 3507 RAND L ET AL ' Reactions catalyzed by potassiun fluoride. III. The Knoevenagel Reaction'
- 'ORGANICUM' 1977 , VEB DEUTSCHER VERLAG DER WISSENSCHAFT , BERLIN 15 AUFLAGE
- ANGEWANDTE CHEMIE Bd. 68, 1956, WEINHEIM,DE Seite 247 LUCIUS G 'CYCLISATION HOMOLOGER SESQUITERPENS[UREN'

## Beschreibung

Die vorliegende Erfindung betrifft ein neues Verfahren zur Herstellung von substituierten Hexensäuren, nämlich der 6-(2',6',6'-Trimethyl-cyclohex-1'-en-1'-yl)-4-methyl-hex-2- oder -3-ensäure (I), welche als Zwischenprodukt für die Herstellung der u.a. unter den Handelsnamen AMBROX DL@ und SYNAMBRAN@ bekannten Riechstoffe geeignet ist. Diese Riechstoffe bestehen im wesentlichen jeweils aus einem Gemisch von (±)-[3aα,5aß,9aα,9bß]-Dodecahydro-3a,6,6,9a-tetramethylnaphtho[2,1-b]furan und (±)-[3aα,5aß,9aα,9bα]-Dodecahydro-3a,6,6,9a-tetramethylnaphtho[2,1-b]furan und sind geeignete Ersatzprodukte für das teure optisch aktive (-)-[3aR-(3aa,5aß,9aa,9bß)]-Dodecahydro-3a,6,6,9a-tetramethylnaphtho[2,1-b]furan, das auf dem Markt unter verschiedenen Handelsnamen käuflich ist, und zwar AMBROX@ (Firmenich), AMBRO-XAN@ (Henkel), AMBERLYN® (Quest), SYLVAMBER® (BASF) sowie AMBROXID® (Haarman & Reimer).

Ferner betrifft die vorliegende Erfindung ein Verfahren zur Herstellung des oben erwähnten Gemisches von [3aa,5aß,9aa,9bß]-Dodecahydro-3a,6,6,9a-tetramethylnaphtho[2,1-b]furan und [3aα,5aß,9aα,9bα]-Dode- cahydro-3a,6,6,9a-tetramethylnaphtho[2,1-b]furan sowie neue Zwischenprodukte in diesem Verfahren.

Es sind bereits einige Verfahren zur Herstellung von AMBROX@ bekannt, welche von Sclareolid, d.h. [3aR-(3aα,5aβ,9aα,9bβ)]-Decahydro-3a,6,6,9a-tetramethylnaphtho[2,1-b]furan-2(1 H)on, ausgehen. Sclareolid selber wird durch Oxidation, beispielsweise unter Verwendung von Chromsäure, von Sclareol (Muskateller Salbeiöl), und Sclareol seinerseits wird aus dem Naturstoff Salvia sclarea durch Extrahieren gewonnen. Nun ist aber die auf dem Markt erhältliche Menge Sclareol beschränkt, und je nach Ernte von Salvia sclarea kann der Preis gewaltig schwanken. Einen weiteren Nachteil dieser Verfahren stellt die vom Umweltschutz her problematische Oxidationsstufe von Sclareol zu Sclareolid mit Chromsäure dar.

Es bestand somit ein Bedürfnis nach einem technisch realisierbaren Verfahren zur Herstellung des Dodecahydro-3a,6,6,9a-tetramethylnaphtho[2,1-b]furans, das nicht über Sclareol erfolgt und demzufolge nicht von der Verfügbarkeit von Sclareol abhängt und zudem wirtschaftlicher und umweltfreundlicher als die bisher verwendeten Verfahren ist. Dies ist nunmehr mittels des erfindungsgemässen Verfahrens möglich.

Dieses Verfahren ist dadurch gekennzeichnet, dass man eine Verbindung der allgemeinen Formel 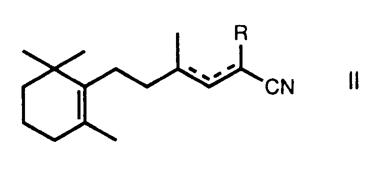 worin eine der Bindungen eine einfache Bindung und die andere eine Doppelbindung bezeichnet und R Wasserstoff oder eine Estergruppe COOR¹ bedeutet, worin R¹ für Niederalkyl oder Aryl steht, verseift und, im Falle, dass R der Formel II eine Estergruppe COOR¹ bedeutet, decarboxyliert, und dass man gewünschtenfalls die so erhaltene(n) Verbindung(en) der Formel 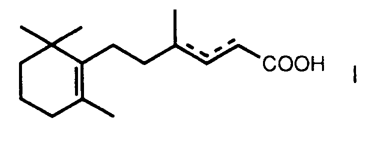 worin die Bindungen die oben angegebene Bedeutung besitzen, [d.h. 6-(2',6',6'-Trimethyl-cyclohex-1'-en-1'-yl)-4-methyl-hex-2-ensäure bzw. 6-(2',6',6'-Trimethyl-cyclohex-1'-en-1'-yl)-4-methyl-hex-3-ensäure] in bekannter Weise in ein Gemisch von [3aα,5aβ,9aα,9bβ]-Dodecahydro-3a,6,6,9a-tetramethylnaphtho[2,1-b]furan und [3aa,5aß,9aa,9ba]-Dodecahydro-3a,6,6,9a-tetramethylnaphtho-[2,1-b]furan überführt.

Im obigen und im folgenden wird die in der Chemie, insbesondere der Carotinoid-Chemie, übliche abgekürzte Darstellung der Strukturformeln benutzt: aliphatische Ketten und Cyclohexenringe, und selbst Methylsubstituente, werden durch einfache Striche wiedergegeben.

In der Definition der Verbindungen der Formel II ist unter "Niederalkyl" (R¹) ein 1 bis 6 Kohlenstoffatome enthaltender, geradkettiger oder verzweigter Alkylrest zu verstehen, wie beispielsweise Methyl, Aethyl, n-Propyl, Isopropyl, n-Butyl, tert.Butyl, n-Pentyl, Neopentyl und n-Hexyl. "Aryl" bedeutet im Rahmen der vorliegenden Erfindung insbesondere eine gegebenenfalls mit Methyl mono- oder mehrfach substituierte Phenylgruppe, wie beispielsweise Phenyl, o-, m- oder p-Tolyl, und die Xylyle.

Durch das Vorliegen der aliphatischen C = C-Doppelbindung in den Verbindungen der Formeln I und II tritt geometrische Isomerie auf, d.h. die Verbindung 1 oder II liegt in der cis-(Z-) oder der trans-(E-)Form vor. Zudem hat das mögliche Vorhandensein eines asymmetrischen Kohlenstoffatoms zur Folge, dass die Verbindungen II in optisch isomeren Formen auftreten können. Wenn nicht anderweitig dargestellt, soll die Formel 1 bzw. 11 die möglichen isomeren Formen sowie Isomerengemische umfassen.

Die Verseifung der Verbindung der allgemeinen Formel II und, falls angebracht, die nachträgliche Decarboxylierung, kann zweckmässigerweise durchgeführt werden, indem man die Verbindung der Formel II in einem inerten protischen organischen Lösungsmittel, wie einem Alkohol, z.B. Aethanol oder Isopropanol, oder einem Glykol, z.B. Mono-, Di- oder Triäthylenglykol; in einem aromatischen Kohlenwasserstoff, z.B. Benzol, Toluol oder einem Xylol; in einem cyclischen Aether, z.B. Tetrahydrofuran oder Dioxan; oder in Wasser mit einer Base bei Temperaturen zwischen etwa 20 °C und etwa 130 °C, insbesondere bei der Rückflusstemperatur des Reaktionsgemisches, behandelt. Als Basen kommen vorzugsweise Alkalimetallhydroxide in Betracht, wobei Natronlauge und Kaliumhydroxid besonders bevorzugte Basen darstellen. Bedeutet R der Formel II eine Estergruppe, so wird diese unter den Reaktionsbedingungen in die Carboxygruppe umgewandelt, die ihrerseits dann decarboxyliert wird. Nach erfolgter Reaktion, die mehrere Stunden dauern kann, wird aufgearbeitet, und zwar auf an sich bekannte Weise, z.B. durch Ansäuern des Gemisches mit Schwefelsäure, Extrahieren mit einem mit Wasser nicht mischbaren organischen Lösungsmittel, wie beispielsweise Diäthyläther, Reinigung der organischen Phase und Abdampfen des organischen Lösungsmittels. Näheres zu diesem Reaktionstyp ist beispielsweise in Organikum, 17. Aufl., 1988, Seite 424, beschrieben.

Sofern keine gezielte Synthese zur Isolierung reiner Isomerer durchgeführt wird, kann das Produkt als Gemisch zweier oder mehrerer Isomerer anfallen. Dies geschieht ohnehin, wenn das Ausgangsmaterial der Formel II als Isomerengemisch vorliegt. Falls gewünscht können die so hergestellten Isomeren nach an sich bekannten Methoden aufgetrennt werden, wie fraktionierte Kristallisation oder Säulenchromatographie. In der Praxis ist dies jedoch üblicherweise nicht nötig, da das Reaktionsprodukt in einem mehrstufigen Verfahren in den erwünschten Riechstoff übergeführt wird und die Auftrennung im Verlauf dieser Umwandlung durchgeführt wird.

Die Ueberführung der Verbindung der Formel I in das Gemisch von [3aa,5aß,9aa,9bß]-Dodecahydro-3a,6,6,9a-tetramethylnaphtho[2,1-b]furan und [3aa,5aß,9aa,9ba]-Dodecahydro-3a,6,6,9a-tetramethylnaphtho-[2,1-b]-furan kann in an sich bekannter Weise erfolgen, beispielsweise durch Cyclisierung der entsprechenden Verbindung 1 oder des Gemisches der beiden Verbindungen I (wobei die jeweilige Verbindung I selber als reines Isomere oder als Isomerengemisch vorliegt) zu Sclareolid und gegebenenfalls weiteren Isomeren, wie beispielsweise Episclareolid und Isosclareolid, Reduktion des Cyclisierungsproduktes zum entsprechenden Diol(gemisch) und schliesslich Dehydratisierung des Diolproduktes. Ein typisches mehrstufiges Verfahren für die Ueberführung der Verbindung(en) der Formel I in das Gemisch von [3aa,5aß,9aa,9bß]-Dodecahydro-3a,6,6,9a-tetramethy!naphtho[2,1-b]furan und [3aα,5aβ,9aα,9bα]-Dodecahydro-3a,6,6,9a-tetramethylnaphtho[2,1-b]furan wird im nachfolgenden Reaktionsschema dargestellt, in dem die Zeichen 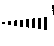 " 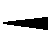 " und "" bedeuten, dass sich der entsprechende Rest oberhalb resp. unterhalb der Molekülebene befindet.

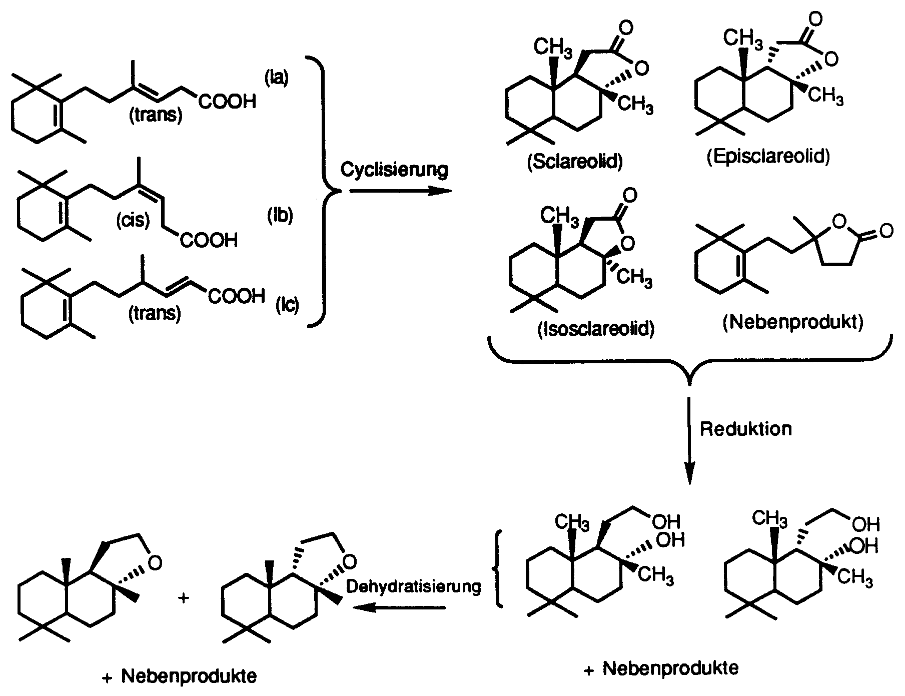

Gemäss Lucius [Archiv der Pharmazie 291, 1958, 1058, 55; Chem. Ber. 1960, 2663; Angew. Chem. 68, 247, 1956; sowie DDR-Patentschrift 13535] kann die Cyclisierung mit einem Gemisch von Ameisensäure und Schwefelsäure bewerkstelligt werden, wobei allerdings die genaue Stereochemie der so erhaltenen drei Laktone nicht eindeutig charakterisiert werden konnte. A Saito et al. (Chem. Lett. 1981, 757-760 sowie Chem. Lett. 1983, 729-732) haben die Cyclisierung genau untersucht und die genaue Stereochemie dieser Laktone zugeordnet. Die Durchführung dieser Cyclisierung besteht darin, dass man die E-6-(2',6',6'-Trimethyl-cyclohex-1'-en-1'-yl)-4-methyl-hex-3-ensäure (la) mit Zinntetrachlorid in Methylenchlorid bei -78 °C behandelt, was ein Gemisch von (±) Sclareolid und (±) Episclareolid in einem Gewichtsverhältnis von ca. 8:1 ergibt. Behandlung von Z-6-(2',6',6'-Trimethyl-cyclo-hex-1'-en-1'-yl)-4-methyl-hex-3-ensäure (Ib) mit Zinntetrachlorid in Methylenchlorid bei -20 °C ergibt nur Episclareolid. Die Cyclisierung mit Zinntetrachlorid wurde zuerst von A. Saito et al. (ibid.) beschrieben (siehe auch H. Kaneko et al., Japanische Patentpublikation 84.014475). Die Cyclisierung kann auch anstatt mit Zinntetrachlorid mit Trifluoressigsäure in Methylenchlorid bei 0°C ausgeführt werden [siehe Europäische Patentpublikation 165.458 sowie T. Kawanobe et al., Agr. Biol. Chem. 50(6), 1986, 1475]; letztere Autoren beschreiben auch die Umsetzung der untenstehenden Verbindung 111 mit Malonsäure zu der oben erwähnten 6-(2',6',6'-Trimethylcyclohex-1'-en-1'-yl)-4-methyl- hex-3-ensäure.

Die Reduktion des Cyclisierungsproduktes (Sclareolid und eventuell weitere Laktone) erfolgt zweckmässigerweise u.a. unter Verwendung von Natrium-bis(2-methoxyäthoxy)aluminiumdihydrid (Redal) als Reduktionsmittel (siehe die Europäische Patentpubtikation 165.458). Weitere Reduktionsmethoden sind beispielsweise aus Maslo-Zhir Prom. st 7, 29-30, 1980 (Chem. Abs. 94, 15913 q), Maslo-Zhir Prom. st. 12, 1979, 25-26 (Chem. Abs. 93, 114751 w) und der UdSSR-Patentschrift 559.916 (Chem. Abs. 87, 136064 c) bekannt geworden.

Die Herstellung des Gemisches von [3aa,5aß,9aa,9bß]-Dodecahydro-3a,6,6,9a-tetramethylnaphtho[2,1-b]furan und [3aa,5aß,9aa,9ba]-Dodecahydro-3a,6,6,9a-tetramethylnaphtho[2,1-b]furan aus dem Diol-(gemisch) durch Dehydratisierung kann beispielsweise unter Verwendung von Naphthalen-ß-sulfonsäure [siehe z.B. Stoll et al., Helv. Chim. Acta 33, 1950, 1251, Schweizerische Patentschrift 299.369 und U.S. Patentschrift 2809996], p-Toluolsulfonsäure [USSR-Patentschrift 345.153, Byul. Izobret 22, 1972, 94, Chem. Abs. 78, 111554p und Europäische Patentpublikation 170.955], Schwefelsäure [Austral. J. Chem. 24, , 1971, 583, Chem. Abs. 74, 76550c], Aluminiumoxid [siehe obige Referenzen betreffend Naphthalen-ß-sulfonsäure sowie Japanische Patentpublikation 61.033184], Toluolsulfonsäurechlorid [Europäische Patentpublikation 165.458, Austral. J. Chem. 24, 1971, 583 sowie Chem. Abs. 74, 76550c], Phosphoroxychlorid [Deutsche Offenlegungsschrift 3240054] und Dimethylsulfoxid/Trimethylsilylchlorid [Synthesis 1983, 219] als Katalysator durchgeführt werden.

Die Ausgangsmaterialien der Formel 11 sind neu und ebenfalls Gegenstand der vorliegenden Erfindung. Diese Verbindungen können dadurch hergestellt werden, dass man das 4-(2',6',6'-Trimethyl-cyclohex-1'-en-1'-yl)-2-methyl-butanal, d.h. die Verbindung der Formel 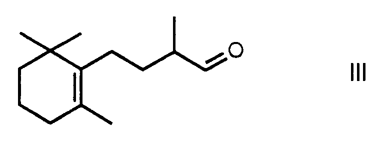 mit Cyanessigsäure oder einem Niederalkyl- oder Arylester davon, d.h. mit einer Verbindung der allgemeinen Formel 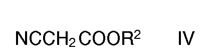 worin R² Wasserstoff, Niederalkyl oder Aryl bedeutet, behandelt.

In der obigen Definition ist unter "Niederalkyl" (R²) insbesondere ein 1 bis 6 Kohlenstoffe enthaltender, geradkettiger oder verzweigter Alkylrest zu verstehen, wie dieser in bezug auf R¹ oben näher erläutert ist. "Aryl" bedeutet insbesondere eine gegebenenfalls mit Methyl mono- oder mehrfach substituierte Phenylgruppe, wie diese ebenfalls in bezug auf R¹ oben näher erläutert ist.

Bei dieser Umsetzung handelt es sich um eine Knoevenagel-Kondensation, die zweckmässigerweise unter Verwendung eines organischen Lösungsmittels sowie einer geringen Menge einer schwachen Base oder alternativ einer Lewis-Säure durchgeführt werden kann. Als Lösungsmittel eignen sich aromatische Kohlenwasserstoffe, z.B. Benzol, Toluol und die Xylole; Alkohole, z.B. Methanol und Aethanol; und aliphatische oder cyclische Aether, z.B. Diäthyläther, Tetrahydrofuran und Dioxan. Beispiele geeigneter Basen sind Ammoniak und primäre, sekundäre und tertiäre Amine, insbesondere Mono- Di- und Triäthylamin, Piperidin, Pyridin, Chinolin und Anilin, sowie Salze davon, insbesondere Ammoniumacetat und Pyridiniumacetat [siehe u.a. Organic Reactions (N.Y.), 15, 1967, 204-599 für Beispiele der Knoevenagel-Reaktion mit solchen Basen]. Falls die Reaktion mit einer Lewis-Säure katalysiert wird, kommt als Katalysator vorzugsweise Kaliumfluorid oder Kaliumjodid in Frage [siehe u.a. L. Rand et al., J. Org. Chem. 1962, 3505; und J.H. Clark, Chem. Rev. 80, 1980, 429-452]. Die Umsetzung erfolgt zweckmässigerweise bei Temperaturen zwischen Raumtemperatur und etwa 180°C, insbesondere bei Raumtemperatur oder bei der Rückflusstemperatur des Reaktionsgemisches (etwa 70 bis 1100 C).

Die erhaltenen Verbindungen der Formel 11 können nach an sich bekannten Methoden isoliert, gereinigt und nötigenfalls in die einzelnen Isomeren aufgetrennt werden.

Besonders bevorzugte Verbindungen der Formel 11 sind 6-(2',6',6'-Trimethyl-cyclohex-1'-en-1'-yl)-4-methyl-hex-3-ennitril sowie die Methyl-, Aethyl-, Isopropyl-, n-Butyl- und tert.Butylester der 6-(2',6',6'-Trimethyl-cyclohex-1'-en-1'-yl)-4-methyl-2-cyano-hex-2-ensäure.

Die beiden Ausgangsmaterialien der Formeln III und IV sind bekannte Verbindungen.

Die nachfolgenden Beispiele dienen zur näheren Erläuterung der Erfindung.

### Beispiel 1

### A. Herstellung von 6-(2',6',6'-Trimethyl-cyclohex-1'-en-1'-yl)-4-methyl-hex-2/3-ennitril

Zu einer Lösung von 120 ml Pyridin, 6,4 ml Piperidin und 48 g (0,56 Mol) Cyanessigsäure in 260 ml Toluol wird bei Rückflusstemperatur eine Lösung von 100 g (0,48 Mol) 4-(2',6',6'-Trimethyl-cyclohex-1'-en-1'-yl)-2-methyl-butanal in 130 ml Toluol rasch zugegeben. Man rührt das Gemisch 6 Stunden bei Rückflusstemperatur unter Abscheidung des in der Reaktion entstehenden Wassers und anschliessend ca. 16 Stunden bei Raumtemperatur. Dann wird das Toluol abdestilliert, Wasser zugegeben, mit Diäthyläther extrahiert und die organische Phase der Reihe nach mit 2N Salzsäure, 2N Natronlauge und Wasser gewaschen, über wasserfreiem Magnesiumsulfat getrocknet und unter vermindertem Druck zur Trockene eingedampft. Auf diese Weise erhält man 100 g (90% der theoretischen Ausbeute) eines Isomerengemisches, das einen Siedepunkt im Bereich von ca. 89°C bis ca. 126°C aufweist und nach Gaschromatographie aus folgenden Isomeren besteht:
(a) Z-6-(2',6',6'-Trimethyl-cyclohex-1'-en-1'-yl)-4-methyl-hex-2-ennitril (2,5%);
(b) E-6-(2',6',6'-Trimethyl-cyclohex-1'-en-1'-yl)-4-methyl-hex-2-ennitril (2,2%);
(c) Z-6-(2',6',6'-Trimethyl-cyclohex-1'-en-1'-yl)-4-methyl-hex-3-ennitril (23%); sowie
(d) E-6-(2',6',6'-Trimethyl-cyclohex-1'-en-1'-yl)-4-methyl-hex-3-ennitril (70%).

### Spektroskopische Daten:-

Isomer (a): ¹H-NMR (CDCl₃; 400 MHz) 6,295 ppm [dd, J =10,8, J =10,8, H-C(3)], 5,295 ppm [d, J =10,8, H-C(2)], 1,56 ppm [s, CH₃-C(2')], 1,09 ppm [d, J = 7, CH₃-C(4)], 1,06 ppm [s, (CH₃)₂-C(6')]; Massenspektrum (m/e): 231(18), 216(98), 199(6,2), 188(5), 175(14), 160(13), 123(100), 107(28), 95(66), 81 (71 67(38), 55(38), 41 (63).
Isomer (b): ¹H-NMR (CDCl₃ ; 400 MHz) 6,66 ppm [dd, J = 8, J = 17, H-C(3)], 5,315 ppm [dd, J =17, J=1, H-C(2)], 1,555 ppm [s, CH₃-C(2')], 1,08 ppm (d, J = 7, CH₃-CH(4)], 0,975 ppm [s, (CH₃)₂-C(6')]; Massenspektrum (m/e): 231 (13), 216(40), 199(2), 188(3), 175(6,2), 160(5), 123(100), 107-(16), 95(37), 81(51), 67(41), 55(34), 41(25).
Isomer (c): Infrarot-Spektrum 2250 cm⁻¹; ¹H-NMR (CDCl₃ ; 400 MHz) 5,14 ppm [ddd, J = 7, J = 7, J = 1,4, H-C(3)], 3,065 ppm [dd, J=7, J 1,4, H₂-C(2)], 2,06 ppm [s breit, H₂-C(5)-C(6)-H₂], 1,925 ppm (dd breit, J=7, J = 7, H₂-C(3')], 1,805 ppm [d, J = 1,4, CH₃-C(4)], 1,645 ppm [s, CH₃-C(2')], 1,015 ppm [s, (CH₃)₂-C(6')]; Massenspektrum (m/e) 231(3), 216(3), 175(3), 137(100), 121(7), 109(10), 95(78), 81(77), 67(22), 57(18), 41 (27).
Isomer (d): Infrarot-Spektrum 2250 cm⁻¹; ¹H-NMR (CDCl₃; 400 MHz) 5,195 ppm [dd, J=7, J=7, H-C(3)], 3,05 ppm [d, J=7, H₂-C-(2)], 2,07 ppm [s, H₂-C(5)-C(6)-H_{2]}, 1,91 ppm [dd breit, J = 7, J = 7, H₂-C(3')], 1,715 ppm [d, J=0,8, CH₃-C(4)], 1,595 ppm [s, CH₃-C(2')], 0,99 ppm [s, (CH₃)₂-C(6')]; Massenspektrum (m/e) 231(4), 216(2), 175(3), 137(100), 121(9), 109(9,5), 95(64), 81(56), 67(14), 57(11), 41(17).

### B. Herstellung von 6-(2',6',6'-Trimethyl-cyclohex-1'-en-1'-yl)-4-methyl-hex-2/3-ensäure

300 g eines Gemisches enthaltend 23 Gewichtsprozent Z-6-(2',6',6'-Trimethyl-cyclohex-1'-en-1'-yl)-4-methyl-hex-3-ennitril und 70 Gewichtsprozent des entsprechenden E-Isomeren (gemäss Teil A hergestellt) werden in einer wässrigen Kaliumhydroxidlösung [182,3 g (3,25M) KOH + 220 ml H₂0] gelöst und 1,6 I Aethanol bei Raumtemperatur zugetropft. Das Reaktionsgemisch wird dann 6 Stunden bei Rückflusstemperatur unter Rühren erhitzt. Man destilliert anschliessend das Aethanol ab und nimmt den Rückstand in Diäthyläther auf. Zur Aether-Lösung gibt man Wasser zu, und das Ganze wird unter Rühren und Kühlen tropfenweise mit 2N Salzsäure behandelt. Man extrahiert die getrennte wässrige Phase mit Diäthyläther, wäscht die vereinigten organischen Phasen neutral mit Wasser, trocknet sie über wasserfreiem Magnesiumsulfat und dampft sie unter vermindertem Druck zur Trockene ein. Auf diese Weise erhält man 315 g eines Isomerengemisches, das nach Gaschromatographie aus folgenden Isomeren besteht:
(e) E-6-(2',6',6'-Trimethyl-cyclohex-1'-en-1'-yl)-4-methyl-hex-2-ensäure (3%);
(f) Z-6-(2',6',6'-Trimethyl-cyclohex-1'-en-1'-yl)-4-methyl-hex-3-ensäure (29%); sowie
(g) E-6-(2',6',6'-Trimethyl-cyclohex-1'-en-1'-yl)-4-methyl-hex-3-ensäure (61 %).

### Beispiel 2

### A. Herstellung von 6-(2',6',6'-Trimethyl-cyclohex-1'-en-1'-yl)-4-methyl-2-cyano-hex-2-ensäure-äthylester

50 g (0,24 Mol) 4-(2',6',6'-Trimethyl-cyclohex-1'-en-1'-yl)-2-methyl-butanal werden in 125 ml Aethanol gelöst und 3,5 g Kaliumfluorid dazugegeben. Man tropft dann 27,2 g (240 mMol) Cyanessigsäureäthylester zu und beobachtet dabei eine leichte Erwärmung des Reaktionsgemisches. Das Gemisch wird dann 1 Stunde bei Raumtemperatur gerührt, und anschliessend eingeengt und die festen Anteile abfiltriert. Nach Destillation des Rückstandes unter Hochvakuum (1,5•10⁻⁴ mbar) erhält man 55 g 6-(2',6',6'-Trimethylcyclo- hex-1'-en-1'-yl)-4-methyl-2-cyano-hex-2-ensäure-äthylester, Sdp. 152°C/5•10⁻⁴ Torr; Infrarot-Spektrum: 2230, 1735, 1625, 1260 cm⁻¹; ¹H-NMR (CDCl₃; 200 MHz): 7,48 ppm [d, J=10,5, C(3)-H], 4,32 ppm (ddd, J=7, CH₃-CH₂-0), 2,86 ppm [m, C(4)-H], 1,56 ppm (s, C(2')-CH₃), 1,36 ppm (dd, J=7, CH₃-CH₂-0), 1,16 ppm [d, J=6,5, CH₃-CH(4)], 0,96 ppm [s, (CH₃)₂C(6')]; ¹³C-NMR (CDCl₃; MHz): 168,0 ppm (d), 161,19 ppm (s), 136,3 (s), 127,4 ppm (s), 113,5 ppm (s), 108,4 ppm (s), 62,2 ppm (t), 39,6 ppm (t), 37,7 ppm (d), 36,3 ppm (t), 34,7 ppm (s), 32,5 ppm (t), 28,4 ppm (q), 26,2 ppm (t), 19,7 ppm (q), 19,3 ppm (t), 18,9 ppm (q), 13,9 ppm (q);
Massenspektrum (m/e): 303(19), 288(60), 260(7), 242(40), 230(3,4), 214(9), 175(10), 137(19), 123(100), 107-(21), 95(54), 81(38), 67(21), 55(26,5), 41(37), 28(44).

### B. Herstellung von 6-(2',6',6'-Trimethyl-cyclohex-1'-en-1'-yl)-4-methyl-hex-2/3-ensäure

Eine Lösung von 7 g 6-(2',6',6'-Trimethyl-cyclohex-1'-en-1'-yl)-4-methyl-2-cyano-hex-2-ensäure-äthyle- ster in 20 ml Aethylenglykol wird mit 5,2 g Kaliumhydroxid in 5,2 ml Wasser versetzt. Dann wird das Reaktionsgemisch 22,5 Stunden unter Rühren bei 130°C erhitzt (Innentemperatur 115°C). Man extrahiert die getrennte wässrige Phase mit Diäthyläther und stellt sie durch Zusatz von 10-prozentiger Salzsäure auf pH 7. Nach weiterem Extrahieren der wässrigen Phase mit Diäthyläther trocknet man die organische Phase über wasserfreiem Magnesiumsulfat und dampft sie unter vermindertem Druck zur Trockene ein. Auf diese Weise erhält man 3,39 g (58% der theoretischen Ausbeute) eines Isomerengemisches, das nach Gaschromatographie aus folgenden Isomeren besteht:
(a) E-6-(2',6',6'-Trimethyl-cyclohex-1'-en-1'-yl)-4-methyl-hex-2-ensäure (21 %);
(b) Z-6-(2',6',6'-Trimethyl-cyclohex-1'-en-1'-yl)-4-methyl-hex-3-ensäure (18%); sowie
(c) E-6-(2',6',6'-Trimethyl-cyclohex-1'-en-1'-yl)-4-methyl-hex-3-ensäure (45%).

### Beispiel 3

### Herstellung von 6-(2',6',6'-Trimethyl-cyclohex-1'-en-1'-yl)-4-methyl-2-cyano-hex-2-ensäure-methylester

50 g (0,24 Mol) 4-(2',6',6'-Trimethyl-cyclohex-1'-en-1'-yl)-2-methyl-butanal werden in 125 ml Aethanol gelöst und 3,5 g Kaliumfluorid dazugegeben. Man tropft dann bei 15°C innert 15 Minuten 23,8 g (0,24 Mol) Cyanessigsäuremethylester zu und rührt das Reaktionsgemisch 1,5 Stunden bei Raumtemperatur nach. Anschliessend wird das Gemisch eingeengt und die festen Anteile abfiltriert. Nach Destillation des Rückstandes (69,7 g) unter Hochvakuum (2 x 10-⁵ mbar) im Kugelrohr erhält man 49,3 g 6-(2',6',6'-Trimethyl-cyclohex-1'-en-1'-yl)-4-methyl-2-cyano-hex-2-ensäure-methylester, Sdp. 178°C/10⁻⁴ mbar; Infrarot-Spektrum (flüssig): 2230, 1740, 1625 cm⁻¹; ¹H-NMR (CDCl₃, 200 MHz): 7,5 ppm [d, J=11, C(3)-H]; 3,88 ppm (s, C0₂CH₃), 2,86 ppm [m, C(4)], 1,56 ppm [s, CH₃-C(2')], 1,16 ppm [d, J=6,8, CH₃-C(4)], 0,96 ppm [s, (CH₃)₂-C(6')]; Massenspektrum (m/e): 289(17), 274(60), 242(38), 214(14), 197(5), 175(7,7), 152(7), 137-(21), 123(100), 107(28), 95(66), 81(65), 67(42), 55(31),41(47), 29(21,8).

### Beispiel 4

### Herstellung von 6-(2',6',6'-Trimethyl-cyclohex-1'-en-1'-yl)-4-methyl-2-cyano-hex-2-ensäure-isopropylester

Analog dem in Beispiel 3 beschriebenen Verfahren wird aus 4-(2',6',6'-Trimethyl-cyclohex-1'-en-1'-yl)-2-methyl-butanal und Cyanessigsäureisopropylester die Titelverbindung hergestellt.

### Physikalische Daten:-

Sdp. 115°C/2.10⁻⁵ mbar;
Infrarot-Spektrum (flüssig): 2230, 1735, 1625 cm⁻¹;
¹H-NMR (CDCl₃, 200 MHz): 7,46 ppm [d, J=11, C(3)-H], 5,14 ppm [m, J=6,4, CH(CH₃)₂], 2,85 ppm [m, C-(4)-H], 1,56 ppm [s, CH₃-C(2')], 1,34 ppm (d, J=6,4, CH₃CHCH₃), 1,33 ppm [d, J=6,4, CH₃CHCH_{3]}, 1,16 ppm [d, J=6,8, CH₃-C(4)], 0,98 ppm [s, (CH₃)₂-C(6')];
Massenspektrum (m/e): 317(15), 302(32), 275(10), 260(56), 242(40), 214(10), 201(4,2), 191(8), 178(13), 137-(32), 123(100), 107(25), 95(56), 81 (60), 69(29), 55(28), 43(50), 28(47).

### Beispiel 5

### Herstellung von 6-(2',6',6'-Trimethyl-cyclohex-1'-en-1'-yl)-4-methyl-2-cyano-hex-2-ensäure-(n-butyl)ester

Analog dem in Beispiel 3 beschriebenen Verfahren wird aus 4-(2',6',6-Trimethyl-cyclohex-1'-en-1'-yl)-2-methyl-butanal und Cyanessigsäure-(n-butyl)ester die Titelverbindung hergestellt.

### Physikalische Daten:-

Sdp. 120 °C/2•20⁻⁵ mbar;
Infrarot-Spektrum (flüssig): 2230, 1730, 1625 cm⁻¹;
¹H-NMR (CDCl₃, 200 MHz): 7,47 ppm (d, J =10,5, C(3)-H), 4,26 ppm (ddd, J = 6,6, J = 6,6, J = 1, -O-CH₂), 2,86 ppm [m, C(4)-H], 1,56 ppm [s, CH₃-C(2')], 1,16 ppm [d, J=7, CH₃-C(4)], 0,96 ppm [s, (CH₃)₂-C(6')]; Massenspektrum (m/e): 331(13), 316(42), 288(28), 274(4), 267(11), 260(17), 242(45), 214(14), 175(14), 137-(25), 123(100), 107(29), 95(65), 81(65), 69(33), 55(45), 41(62), 28(31).

### Beispiel 6

### Herstellung von 6-(2',6',6'-Trimethyl-cyclohex-1'-en-1'-yl)-4-methyl-2-cyano-hex-2-ensäure-tert.butylester

Analog dem in Beispiel 3 beschriebenen Verfahren wird aus 4-(2',6',6'-Trimethyl-cyclohex-1'-en-1'-yl)-2-methyl-butanal und Cyanessigsäure-tert.-butylester die Titelverbindung hergestellt.

### Physikalische Daten:-

Sdp. 120°C/2.10⁻⁵ mbar;
Infrarot-Spektrum (flüssig): 2230, 1735, 1625 cm⁻¹;
¹H-NMR (CDCl₃, 200 MHz): 7,38 ppm [d, J=11, C(3)-H], 2,83 ppm [m, C(4)-H], 1,55 ppm [s, C(CH₃)₂], 1,15 ppm [dd, J=6,5, CH₃-CH(4)], 0,97 ppm [s, (CH₃)₂C(6')];
Massenspektrum (m/e): 331(2), 275(24), 274(41), 260(40), 242(12), 231(4), 216(7), 194(10), 175(8), 137(27), 123(51), 107(18), 95(42), 81 (50), 69(24), 87(85), 41(100), 28(60).

## Patentansprüche (Patentansprüche für folgende(n) Vertragsstaat(en) : CH, DE, FR, GB, IT, LI, NL)

1. Verfahren zur Herstellung von 6-(2',6',6'-Trimethyl-cyclohex-1'-en-1'-yl)-4-methyl-hex-2- oder -3-ensäu- re bzw. eines Gemisches von [3aa,5aß, 9aa,9bß]-Dodecahydro-3a,6,6,9a-tetramethylnaphtho[2,1-b]-furan und [3aα,5aβ,9aα,9bα]Dodecahydro-3a,6,6,9-tetramethylnaphtho[2,1-b]furan, dadurch gekennzeichnet, dass man eine Verbindung der allgemeinen Formel 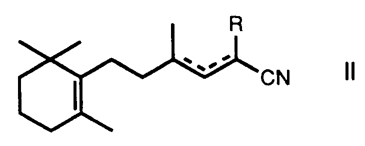 worin eine der Bindungen eine einfache Bindung und die andere eine Doppelbindung bezeichnet und R Wasserstoff oder eine Estergruppe COOR¹ bedeutet, worin R¹ für Niederalkyl mit 1 bis 6 C-Atomen oder Aryl steht,
verseift und, im Falle, dass R der Formel II eine Estergruppe COOR¹ bedeutet, decarboxyliert, und dass man gewünschtenfalls die so erhaltene(n) Verbindung(en) der Formel 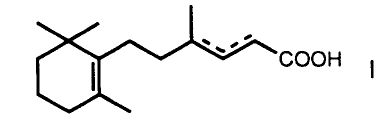 worin die Bindungen -- die oben angegebene Bedeutung besitzen, in bekannter Weise in ein Gemisch von [3aa,5aß,9aa,9bß]-Dodecahydro-3a,6,6,9a-tetramethylnaphtho-[2, 1-b]furan und [3aα,5aβ,9aα,9bα]-Dodecahydro-3a,6,6,9a-tetramethylnaphtho[2,1-b]furan überführt.

2. Verfahren nach Anspruch 1, dadurch gekennzeichnet, dass man eine Verbindung der allgemeinen Formel II in einem inerten protischen organischen Lösungsmittel, in einem aromatischen Kohlenwasserstoff; in einem cyclischen Aether; oder in Wasser mit einer Base bei Temperaturen zwischen etwa 20°C und etwa 130 °C behandelt.

3. Verfahren nach Anspruch 2, dadurch gekennzeichnet, dass man eine Verbindung der allgemeinen Formel II in einem Alkohol, in einem Glykol, in Benzol, Toluol oder einem Xylol, in Tetrahydrofuran oder Dioxan, oder in Wasser mit einem Alkalimetallhydroxid bei der Rückflusstemperatur des Reaktionsgemisches behandelt.

4. Verfahren nach Anspruch 1, 2 oder 3, dadurch gekennzeichnet, dass man die Verbindung II durch Behandlung von 4-(2',6',6'-Trimethyl-cyclohex-1'-en-1'-yl)-2-methyl-butanal mit einer Verbindung der allgemeinen Formel 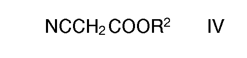 worin R² Wasserstoff, Niederalkyl oder Aryl bedeutet, erhält.

5. Verbindungen der allgemeinen Formel 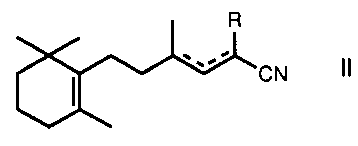 worin eine der Bindungen eine einfache Bindung und die andere eine Doppelbindung bezeichnet und R Wasserstoff oder eine Estergruppe COOR^{I} bedeutet, worin R¹ für Niederalkyl mit 1 bis 6 C-Atomen oder Aryl steht.

6. Eine Verbindung nach Anspruch 5, ausgewählt aus:
6-(2',6',6'-Trimethyl-cyclohex-1'-en-1'-yl)-4-methyl-hex-3-ennitril,
6-(2',6',6'-Trimethyl-cyclohex-1'-en-1'-yl)-4-methyl-2-cyano-hex-2-ensäure-methylester,
6-(2',6',6'-Trimethyl-cyclohex-1'-en-1'-yl)-4-methyl-2-cyano-hex-2-ensäure-äthylester,
6-(2',6',6'-Trimethyl-cyclohex-1'-en-1'-yl)-4-methyl-2-cyano-hex-2-ensäure-isopropylester,
6-(2',6',6'-Trimethyl-cyclohex-1'-en-1'-yl)-4-methyl-2-cyano-hex-2-ensäure-(n-butyl)ester und
6-(2',6',6'-Trimethyl-cyclohex-1'-en-1'-yl)-4-methyl-2-cyano-hex-2-ensäure-tert.butylester.

## Patentansprüche (Patentansprüche für folgende(n) Vertragsstaat(en) : ES)

1. Verfahren zur Herstellung von 6-(2',6',6'-Trimethyl-cyclohex-1'-en-1'-yl)-4-methyl-hex-2- oder -3-ensäu- re bzw. eines Gemisches von [3aa,5aß, 9aa,9bß]-Dodecahydro-3a,6,6,9a-tetramethylnaphtho[2,1-b]-furan und [3aa,5aß,9aa,9ba]-Dodecahydro-3a,6,6,9-tetramethylnaphtho[2,1-b]füran, dadurch gekennzeichnet, dass man eine Verbindung der allgemeinen Formel 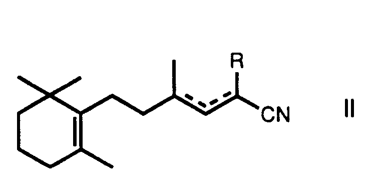 worin eine der Bindungen eine einfache Bindung und die andere eine Doppelbindung bezeichnet und R Wasserstoff oder eine Estergruppe COOR^{I} bedeutet, worin R¹ für Niederalkyl mit 1 bis 6 C-Atomen oder Aryl steht,
verseift und, im Falle, dass R der Formel II eine Estergruppe COOR^{I} bedeutet, decarboxyliert, und dass man gewünschtenfalls die so erhaltene(n) Verbindung(en) der Formel 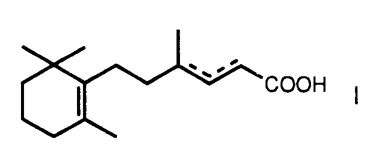 worin die Bindungen die oben angegebene Bedeutung besitzen,
in bekannter Weise in ein Gemisch von [3aa,5aß,9aa,9bß]-Dodecahydro-3a,6,6,9a-tetramethylnaphtho-[2,1-b]furan und [3aα,5aβ,9aα,9bα]-Dodecahydro-3a,6,6,9a-tetramethylnaphtho[2,1-b]furan überführt.

2. Verfahren nach Anspruch 1, dadurch gekennzeichnet, dass man eine Verbindung der allgemeinen Formel II in einem inerten protischen organischen Lösungsmittel, in einem aromatischen Kohlenwasserstoff; in einem cyclischen Aether; oder in Wasser mit einer Base bei Temperaturen zwischen etwa 20°C und etwa 130°C behandelt.

3. Verfahren nach Anspruch 2, dadurch gekennzeichnet, dass man eine Verbindung der allgemeinen Formel II in einem Alkohol, in einem Glykol, in Benzol, Toluol oder einem Xylol, in Tetrahydrofuran oder Dioxan, oder in Wasser mit einem Alkalimetallhydroxid bei der Rückflusstemperatur des Reaktionsgemisches behandelt.

4. Verfahren nach Anspruch 1, 2 oder 3, dadurch gekennzeichnet, dass man die Verbindung II durch Behandlung von 4-(2',6',6'-Trimethyl-cyclohex-1'-en-1'-yl)-2-methyl-butanal mit einer Verbindung der allgemeinen Formel 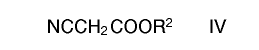 worin R² Wasserstoff, Niederalkyl mit 1 bis 6 C-Atomen oder Aryl bedeutet, erhält.

## Claims (Claims for the following Contracting State(s) : CH, DE, FR, GB, IT, LI, NL)

1. A process for the manufacture of 6-(2',6',6'-trimethyl-cyclohex-1'-en-1'-yl)-4-methyl-hex-2- or -3-enoic acid and of a mixture of [3aα,5aβ, 9aα,9bα]-dodecahydro-3a,6,6,9a-tetramethyl-naphtho[2,1-b]furan and [3aα,5aβ,9aα,9bα]-dodecahydro-3a,6,6,9-tetramethylnaphtho[2,1-b]furan, characterized by saponifying a compound of the general formula 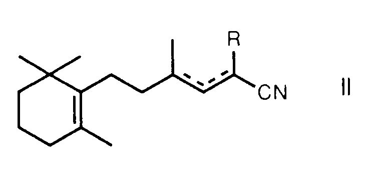 wherein one of the bonds denotes a single bond and the other denotes a double bond and R signifies hydrogen of an ester group COOR¹ in which R¹ stands for lower alkyl with 1 to 6 C atoms or aryl,
and, where R in formula II signifies an ester group COOR¹, decarboxylating, and, if desired, converting the thus-obtained compound(s) of the formula 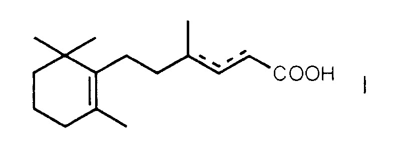 wherein the bonds have the significance given above, in a known manner into a mixture of [3aα,5aβ,9aα,9bβ]-dodecahydro-3a,6,6,9a-tetramethylnaphtho[2,1-b]furan and [3aα,5aβ, 9aα,9bβ]-dodecahydro-3a,6,6,9a-tetramethylnaphtho[2,1-b]furan.

2. A process according to claim I, characterized in that a compound of general formula II is treated in an inert protic organic solvent, in an aromatic hydrocarbon; in a cyclic ether; or in water with a base at temperatures between about 20 °C and about 130°C.

3. A process according to claim 2, characterized in that a compound of general formula II is treated in an alcohol, in a glycol, in benzene, toluene or a xylene, in tetrahydrofuran or dioxan or in water with an alkali metal hydroxide at the reflux temperature of the reaction mixture.

4. A process according to claim 1, 2 or 3, characterized in that the compound II is obtained by treating 4-(2',6',6'-trimethyl-cyclohex-1'-en-1'-yl)-2-methyl-butanal with a compound of the general formula 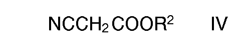 wherein R² signifies hydrogen, lower alkyl or aryl.

5. Compounds of the general formula 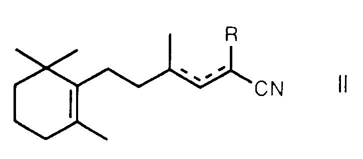 wherein one of the bonds denotes a single bond and the other denotes a double bond and R signifies hydrogen or an ester group COOR¹ in which R¹ stands for lower alkyl with 1 to 6 C atoms or aryl.

6. A compound according to claim 5, selected from:
6-(2',6',6'-Trimethyl-cyclohex-1'-en-1'-yl)-4-methyl-hex-3-enenitrile,
methyl 6-(2',6',6'-trimethyl-cyclohex-1'-en-1'-yl)-4-methyl-2-cyano-hex-2-enoate,
ethyl 6-(2',6',6'-trimethyl-cyclohex-1'-en-1'-yl)-4-methyl-2-cyano-hex-2-enoate
isopropyl 6-(2',6',6'-trimethyl-cyclohex-1'-en-1'-yl)-4-methyl-2-cyano-hex-2-enoate,
n-butyl 6-(2',6',6'-trimethyl-cyclohex-1'-en-1'-yl)-4-methyl-2-cyano-hex-2-enoate and
tert.butyl 6-(2',6',6'-trimethyl-cyclohex-1'-en-1'-yl)-4-methyl-2-cyano-hex-2-enoate.

## Claims (Claims for the following Contracting State(s) : ES)

1. A process for the manufacture of 6-(2',6',6'-trimethyl-cyclohex-1'-en-1'-yl)-4-methyl-hex-2- or -3-enoic acid and of a mixture of [3aα,5aβ,9aα,9bβ]-dodecahydro-3a,6,6,9a-tetramethyl-naphtho[2,1-b]furan and [3aα,5aβ,9aα,9bα]-dodecahydro-3a,6,6,9-tetramethylnaphtho[2,1-b]furan, characterized by saponifying a compound of the general formula 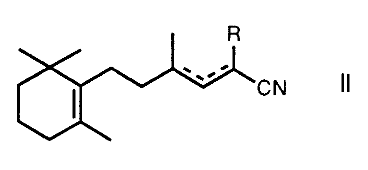 wherein one of the bonds denotes a single bond and the other denotes a double bond and R signifies hydrogen of an ester group COOR¹ in which R¹ stands for lower alkyl with 1 to 6 C atoms or aryl,
and, where R in formula II signifies an ester group COOR¹, decarboxylating,
and, if desired, converting the thus-obtained compound(s) of the formula 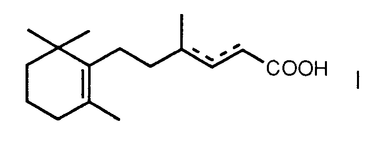 wherein the bonds 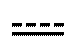 have the significance given above, in a known manner into a mixture of [3aα,5aβ,9aα,9bβ]-dodecahydro-3a,6,6,9a-tetramethylnaphtho[2,1-b]furan and [3aα,5aβ, 9aα,9bα]-dodecahydro-3a,6,6,9a-tetramethylnaphtho[2,1-b]furan.

2. A process according to claim I, characterized in that a compound of general formula II is treated in an inert protic organic solvent, in an aromatic hydrocarbon; in a cyclic ether; or in water with a base at temperatures between about 20°C and about 130°C.

3. A process according to claim 2, characterized in that a compound of general formula II is treated in an alcohol, in a glycol, in benzene, toluene or a xylene, in tetrahydrofuran or dioxan or in water with an alkali metal hydroxide at the reflux temperature of the reaction mixture.

4. A process according to claim 1, 2 or 3, characterized in that the compound II is obtained by treating 4-(2',6',6'-trimethyl-cyclohex-1'-en-1'-yl)-2-methyl-butanal with a compound of the general formula 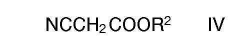 wherein R² signifies hydrogen, lower alkyl with 1 to 6 C atoms or aryl.

## Revendications (Revendications pour l'(les) Etat(s) contractant(s) suivant(s) : suivants : CH, DE, FR, GB, IT, LI, NL)

1. Procédé de préparation de l'acide 6-(2',6',6'-triméthylcyclohexa-1'-ène-1'-yl)-4-méthylhexa-2- ou -3- énoïque ou d'un mélange de [3aalpha,5abêta,9aalpha,9bbêta]-dodécahydro-3a,6,6,9a-tétraméthylnaphto[2,1-b]furanne et de [3aalpha,5abêta,9aalpha,9balpha]-dodécahydro-3a,6,6,9-tétraméthylnaphto[2,1-b]furanne, caractérisé en ce que l'on saponifie un composé de formule générale 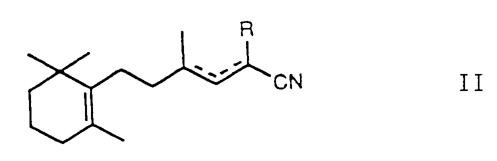 dans laquelle l'une des liaisons est une liaison simple et l'autre une double liaison, et R représente l'hydrogène ou un groupe ester COOR¹ dans lequel R¹ représente un groupe alkyle inférieur en C1-C6 ou aryle, et, lorsque le symbole R de la formule Il représente un groupe ester COOR^{I}, on décarboxyle, et si on le désire, on convertit le ou les composés ainsi obtenus, de formule 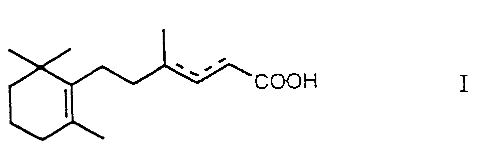 dans laquelle les liaisons sont telles que définies ci-dessus,
de manière connue en soi, en un mélange de [3aalpha,5abêta,9aalpha,9bbêta]-dodécahydro-3a,6,6,9a-tétraméthylnaphto[2,1-b]furanne et de [3aalpha,5abêta,9aalpha,9balpha]-dodécahydro-3a,6,6,9a-tétraméthylnaphto[2,1-b]furanne.

2. Procédé selon revendication 1, caractérisé en ce que l'on traite un composé de formule générale Il dans un solvant protonique inerte, dans un hydrocarbure aromatique ; dans un éther cyclique ; ou dans l'eau, à l'aide d'une base, à des températures allant d'environ 20 à 130°C.

3. Procédé selon revendication 2, caractérisé en ce que l'on traite un composé de formule générale Il dans un alcool, dans un glycol, dans le benzène, le toluène ou un xylène, dans le tétrahydrofuranne ou le dioxanne, ou dans l'eau, à l'aide d'un hydroxyde de métal alcalin à la température de reflux du mélange de réaction.

4. Procédé selon revendication 1, 2 ou 3, caractérisé en ce que l'on obtient le composé II en traitant le 4-(2',6',6'-triméthylcyclohexa-1'-ène-1'-yl)-2-méthylbutanal par un composé de formule générale 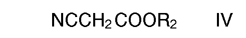 dans laquelle R² représente l'hydrogène, un groupe alkyle inférieur en C1-C6 ou aryle.

5. Composés de formule générale 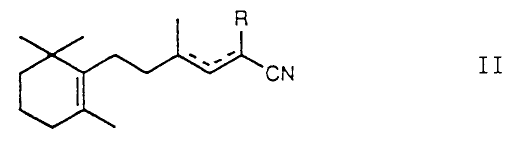 dans laquelle l'une des liaisons est une liaison simple et l'autre une double liaison, et R représente l'hydrogène ou un groupe ester COOR¹ dans lequel R¹ représente un groupe alkyle inférieur en C1-C6 ou aryle.

6. Un composé selon revendication 5, choisi parmi les suivants :
6-(2',6',6'-triméthylcyclohexa-1'-ène-1'-yl)-4-méthylhexa-3-ènenitrile,
6-(2',6',6'-triméthylcyclohexa-1'-ène-1'-yl)4-méthyl-2-cyanohexa-2-énoate de méthyle,
6-(2',6',6'-triméthylcyclohexa-1'-ène-1'-yl)-4'méthyl-2-cyanohexa-2-énoate d'éthyle,
6-(2',6',6'-triméthylcyclohexa-1'-ène-1'-yl)-4-méthyl-2-cyanohexa-2-énoate d'isopropyle,
6-(2',6',6'-triméthylcyclohexa-1'-ène-1'-yl)-4-méthyl-2-cyclohexa-2-énoate de n-butyle et
6-(2',6',6'-triméthylcyclohexa-1'-ène-1'-yl)-4-méthyl-2-cyanohexa-2-énoate de tert-butyle.

## Revendications (Revendications pour l'(les) Etat(s) contractant(s) suivant(s) : suivant : ES)

1. Procédé de préparation de l'acide 6-(2',6',6'-triméthylcyclohexa-1'-ène-1'-yl)-4-méthylhexa-2- ou -3- énoïque ou d'un mélange de [3aalpha,5abêta,9aalpha,9bbêta]-dodécahydro-3a,6,6,9a-tétraméthylnaphto[2,1-b]furanne et de [3aalpha,5abêta,9aalpha,9balpha]-dodécahydro-3a,6,6,9-tétraméthylnaphto[2,1-b]furanne, caractérisé en ce que l'on saponifie un composé de formule générale 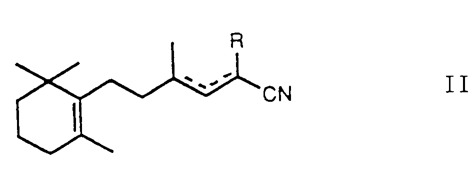 dans laquelle l'une des liaisons est une liaison simple et l'autre une double liaison, et R représente l'hydrogène ou un groupe ester COOR¹ dans lequel R¹ représente un groupe alkyle inférieur en C1-C6 ou aryle, et, lorsque le symbole R de la formule Il représente un groupe ester COOR¹, on décarboxyle, et si on le désire, on convertit le ou les composés ainsi obtenus, de formule 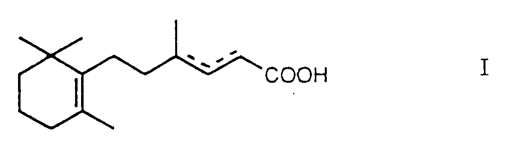 dans laquelle les liaisons sont telles que définies ci-dessus, de manière connue en soi, en un mélange de [3aalpha,5abêta,9aalpha,9bbêta]-dodécahydro-3a,6,6,9a-tétraméthylnaphto[2,1-b]furanne et de [3aalpha-5abêta,9aalpha,9balpha]-dodécahydro-3a,6,6,9a-tétra- méthylnaphto[2,1 -b]furanne.

2. Procédé selon revendication 1, caractérisé en ce que l'on traite un composé de formule générale Il dans un solvant protonique inerte, dans un hydrocarbure aromatique ; dans un éther cyclique ; ou dans l'eau, à l'aide d'une base, à des températures allant d'environ 20 à 130°C.

3. Procédé selon revendication 2, caractérisé en ce que l'on traite un composé de formule générale II dans un alcool, dans un glycol, dans le benzène, le toluène ou un xylène, dans le tétrahydrofuranne ou le dioxanne, ou dans l'eau, à l'aide d'un hydroxyde de métal alcalin à la température de reflux du mélange de réaction.

4. Procédé selon revendication 1, 2 ou 3, caractérisé en ce que l'on obtient le composé II en traitant le 4-(2',6',6'-triméthylcyclohexa-1'-ène-1'-yl)-2-méthylbutanal par un composé de formule générale 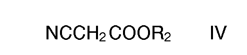 dans laquelle R² représente l'hydrogène, un groupe alkyle inférieur en C1-C6 ou aryle.
